# EUROPEAN PATENT SPECIFICATION

(11) **EP 2 318 100 B1**
(45) Date of publication and mention of the grant of the patent: **21.12.2011**
(21) Application number: 09786981.2
(22) Date of filing: 19.08.2009
(51) Int. Cl.: A61Q 13/00, C11B 9/00, C11D 3/50, A23L 1/226

(54) **EQUILIBRATED DYNAMIC MIXTURES CONTAINING STABILIZED HEMIACETALS FOR THE CONTROLLED RELEASE OF ACTIVE ALCOHOLS**
AUSGEWOGENE DYNAMISCHE MISCHUNGEN MIT STABILISIERTEN HALBACETALEN ZUR GESTEUERTEN FREISETZUNG AKTIVER ALKOHOLE
MÉLANGES DYNAMIQUES ÉQUILIBRÉS CONTENANT DES HÉMIACÉTALS STABILISÉS POUR LA LIBÉRATION CONTRÔLÉE D'ALCOOLS ACTIFS

(30) Priority: 22.08.2008 EP 08162801
(43) Date of publication of application: 11.05.2011
(73) Proprietor: Firmenich S.A., 1211 Geneva 8 (CH); Université de Strasbourg, 67081 Strasbourg Cedex (FR); Centre National de la Recherche Scientifique, 75794 Paris Cedex 16 (FR)
(72) Inventor: LEHN, Jean-Marie, F-67000 Strasbourg (FR); DRAHONOVSKY, Dusan, F-67083 Strasbourg (FR); HERRMANN, Andreas, CH-1273 Le Muids (CH)
(74) Representative: Carina, Riccardo Filippo
(86) International application number: PCT/IB2009/053659
(87) International publication number: WO 2010/020954

(56) References cited:
- EP-A- 1 857 436
- WO-A-01/07095
- US-B1- 6 277 796
- US-B1- 6 395 695

## Description

### Technical field

The present invention concerns a dynamic mixture containing stabilized hemiacetals obtained by combining at least one volatile active alcohol with a composition comprising specific aldehyde derivatives and stabilizing salts. The invention's mixture is capable of releasing in a controlled and prolonged manner said volatile active alcohol to the surrounding environment.

The present invention concerns also the use of said dynamic mixtures as perfuming ingredients as well as the perfuming compositions or perfumed articles comprising the invention's mixtures. A further object of the present invention is a composition comprising said specific aldehyde derivatives and said stabilizing salts as additives to prolong the effect of said volatile active alcohols.

### Prior art

Flavors and fragrances, but also insect attractants or repellents, as well as some bactericides, fungicides or pharmaceuticals, are active volatile molecules that can only be perceived over a limited period of time.

The flavor and fragrance industry has a particular interest for compositions or additives which are capable of prolonging or enhancing the flavoring or perfuming effect of a mixture of several fragrances at the same time over a certain period of time. It is particularly desirable to obtain long-lasting properties for standard raw materials which are too volatile or have a poor substantivity by themselves, or which are only deposited in a small amount onto the surface of the final application. Long-lasting perfumes are desirable for various applications, as for example fine or functional perfumery or cosmetic preparations. Given the importance of this type of problematic in the perfume industry, research in this field has been sustained, in particular with the aim of finding new, and more effective solutions to the aforementioned problems.

One possibility to deliver bioactive compounds is the preparation of precursor compounds which are obtained by covalently attaching the active compound to a suitable substrate or carrier material. A variety of precursor compounds which release active material by a chemical reaction during or after application (using O₂, light, enzymes, water (pH) or temperature as the release trigger) have been described in the prior art (e.g. Angew. Chem. Int. Ed., vol. 46, pages 5836-5863 (2007)) as an alternative to encapsulation systems. In general, due to their inherent instability, the precursors often decompose in the application base during storage and thus release their fragrance raw material before the desired use.

However, in many cases, the covalent attachment of the active compound to a substrate generates precursors (e.g. acetals or ketals) which are too stable to be efficiently decomposed in practical applications. Therefore, there is a strong need to develop delivery systems based on particularly weak chemical bonds that can easily be cleaved under mild environmental conditions.

In WO 2006/016248, the controlled release of fragrances from dynamic mixtures from a hydrazine/hydrazone equilibrium has been reported. Due to the reversibility of the covalent bond formation in aqueous media, the precursors are easily cleaved in practical applications to release volatile, bioactive carbonyl compounds. However said system only allows the controlled release of active aldehydes or ketones, but not of alcohols.

We have now surprisingly found that some stabilized hemiacetals, optionally further stabilized by addition of a stabilizing salt, can be used to improve the release of volatile active alcohols in applications by the in situ formation of dynamic mixtures.

To the best of our knowledge, none of the compositions of the present invention have been described or suggested for the controlled and/or improved delivery of standard (i.e. of current use) volatile active alcohols, in particular perfumery alcohols.

### Description of the invention

We have now surprisingly found that a dynamic mixture containing stabilized hemiacetals, obtainable by combining at least one active volatile alcohol with a composition, comprising at least one specific aldehyde derivative and, optionally, a stabilizing salt, is a valuable ingredient capable of releasing, in a controlled and prolonged manner, said active alcohol.

As "dynamic mixture" we mean here a composition comprising a solvent, several starting components as well as several addition products that are the results of reversible reactions between the various starting components. It is believed that said dynamic mixtures take advantage from reversible chemical reactions, in particular from the formation and dissociation by reversible condensation between the carbonyl group of the aldehyde derivative and the OH group of the active volatile alcohol to be released. The ratio between the various starting and addition products depends on the equilibrium constant of each possible reaction between the starting components. The usefulness of said "dynamic mixture" derives from a synergistic effect between all the components.

By the term "active" we mean here that the volatile alcohol to which it is referred is capable of bringing a benefit or effect into its surrounding environment, and in particular a perfuming, flavoring, malodor counteracting, pharmaceutical, bactericide, fungicide and/or insect repellent or attractant effect. Therefore, for example, said "volatile alcohol" possesses at least one property which renders it useful as perfuming or flavoring ingredient, pharmaceutical composition, bactericide, fungicide and/or as insect repellent or attractant.

By the term "malodor counteracting" we mean here that the volatile alcohol to which it is referred is capable of bringing a benefit or effect into its surrounding environment by reducing the perception of malodor, i.e. of an odor that is unpleasant or offensive to the human nose. Said term is not intended to be limited to any particular mechanism of action, provided that malodor is counteracted.

According to all the above and below mentioned embodiments of the invention, the invention's dynamic mixture is particularly useful when the active volatile alcohol is a perfuming ingredient, i.e. a perfuming alcohol. A "perfuming alcohol" is a compound, which is of current use in the perfumery industry, i.e. a compound which is used as active ingredient in perfuming preparations or compositions in order to impart a hedonic effect. In other words, such a volatile alcohol, to be considered as being a perfuming one, must be recognized by a person skilled in the art of perfumery as being able to impart or modify in a positive or pleasant way the odor of a composition, and not just as having an odor. From now on we will refer to said "active volatile alcohol" also as "perfuming alcohol".

Practically, the invention is carried out exactly in the same manner, independently of the exact properties of the active volatile alcohol. Therefore, it is understood that, even if the invention will be further illustrated herein below with a specific reference to "perfuming alcohol", the below embodiments are also applicable to other active volatile alcohol (i.e. it is possible to replace the expression "perfuming" with "flavoring", "malodor counteracting", "pharmaceutical", "bactericide". "fungicide", "insect attractant" or with "insect repellent" for instance).

As previously mentioned, the invention's dynamic mixture enables a controlled release of an active volatile alcohol, and in particular a perfuming one. Such a behavior makes the invention's dynamic mixture particularly suitable as active ingredient. Consequently, the use of an invention's dynamic mixture as active ingredient is an object of the present invention. In particular it concerns a method to confer, enhance, improve or modify the odor properties of a perfuming composition or of a perfumed article, which method comprises adding to said composition or article an effective amount of an invention's dynamic mixture.

Now, the present invention concerns a use as active ingredient of a dynamic mixture (for the controlled release of active volatile alcohol), said dynamic mixture comprises a stabilized hemiacatal and is obtainable by reacting,
i) at least one active volatile alcohol having a molecular weight comprised between 42 and 230 g/mol and being a perfuming, flavoring, malodor counteracting, pharmaceutical, bactericide, fungicide, insect repellent or attractant ingredient, in particular being selected from the group consisting of C₄₋₁₅ perfuming alcohols;
   with a composition comprising
ii) at least one mono, di or tri aldehyde of formula

   HCO-Q (I)

   wherein Q represents a CHO or (CH₂)ₘCOOH group, with m being 0, 1 or 2, or a group of formula 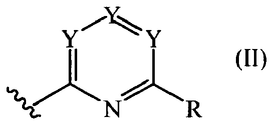 in which R represents a hydrogen atom, a CHO or COOH group or a C₁₋₆ hydrocarbon group, optionally comprising an oxygen or sulfur atom and optionally substituted;
   each Y, simultaneously or independently, represents a CR group, R being defined as above, or a nitrogen atom, provided that two adjacent Y are not simultaneously nitrogen atoms; and two R groups, taken together, may form an unsaturated or aromatic cycle containing 5 or 6 atoms and including the carbon atoms to which said R is bonded, said cycle being substituted or unsubstituted and optionally containing one or two additional nitrogen or oxygen atoms; and,
iii) optionally, a stabilizing salt of formula MXₙ, a stabilizing acid having a pKₐ below 5, or a mixture thereof, wherein n represents 1, 2 or 3, X represents a mono-anion derived from an acid HX having a pKₐ below 6, and M is a cation selected from the group consisting of H⁺, Li⁺, Zn²⁺, Fe²⁺, Fe³⁺, Ni²⁺, Cu²⁺, Y³⁺, Sc³⁺, La³⁺, Ag⁺ and the alkaline-earth cations.

Examples of possible substituents of said R comprise one or two groups such as R¹, OH, OR¹, SR¹, (⁺NR²₃)R³, SO₂R⁴, SO₃R⁴, COOR⁴ or CHO, with R¹ representing a C₁-C₄, hydrocarbon group, or a phenyl group, optionally substituted by the same groups as defined above, R² representing a hydrogen atom or a R¹ group, R³ representing a halogen anion or a sulphate anion, R⁴ representing a hydrogen atom or an alkali metal cation.

According to a particular embodiment of the invention, the dynamic mixtures is obtained by reacting together of at least one volatile active alcohol, at least one specific aldehyde derivative according to formula (I) and at least a stabilizing salt or a stabilizing acid. According to a particular embodiment of the invention a stabilizing salt is preferred to a stabilizing acid.

According to a particular embodiment of the invention, the stabilizing salt is a compound MXₙ, wherein n represents 1, 2 or 3,
M is a cation selected from the group consisting of H⁺, Zn²⁺, Fe³⁺, Cu²⁺, Y³⁺, Sc³⁺, Ca²⁺, Mg²⁺ and Ba²⁺; and
X represents a mono-anion derived from an acid HX having a pKₐ below 5, e.g. comprised between -5 and 5.

According to another particular embodiment of the invention, the stabilizing salt is a compound MXₙ, wherein n represents 1, 2 or 3,
M is a cation selected from the group consisting of H⁺, Zn²⁺, Fe³⁺ and Cu²⁺; and
X represents a mono-anion derived from an acid HX having a pKₐ below 1, e.g. comprised between -5 and 1.

Typical, non-limiting examples of said mono-anion X are: halides (such as Cl⁻, Br⁻ or F⁻), NO₃⁻, H₂PO₃⁻, CO₃⁻, ClO₄⁻, PF₆, SbF₆⁻, R⁵SO₃⁻, R^{5'}COO⁻ wherein R⁵ is a R^{5'} or a chlorine or fluorine atom R^{5'} is a C₁-C₈ alkyl or aryl group or a C₁-C₈ fluoroalkyl or fluoroaryl group (such as C₆F₅SO₃⁻ or CF₃SO₃⁻), BF₄⁻, or BR⁶₄⁻, wherein R⁶ is a phenyl group (Ph) optionally substituted by one to five groups such as halide atoms or methyl or CF₃ groups (such as BPh₄⁻ or B[3,5-(CF₃)₂C₆H₄]₄⁻), or C₁₋₇ carboxylate (such as formiate, acetate, benzoate).

According to a particular embodiment of the invention, the mono-anions X can be an anion such as Cl⁻, Br⁻, F, ClO₄⁻, PF₆⁻, R⁵SO3⁻, R^{5'}COO⁻, BF₄⁻, or BR⁶₄- with R⁵, R^{5'} and R⁶ having the same meaning as described above.

The mono-anion X is ideally chosen to enhance (maximize) the solubility of the stabilizing salt in the solvent in which the dynamic mixtures containing stabilized hemiacetals according to the invention are prepared.

The stabilizing salt might be chosen to have antibacterial or antiperspirant properties, as for example those typically encountered in deodorant or antiperspirant formulations, such as organic or inorganic salts of zinc, copper or mixtures thereof.

According to a particular embodiment of the invention, the stabilizing acid is a compound HₘA, wherein m represents 1, 2 or 3, and A is a non-, di- or tri-anion. Said acid having a pKₐ below 5, e.g. comprised between -5 and 5.

Typical, non-limiting examples of said anion A are: halides (such as Cl⁻, Br⁻ or F), NO₃⁻, H₂PO₄⁻, HPO₄²⁻. PO₄³⁻, SO₄²⁻, HCO₃⁻, CO₃²⁻, ClO₄⁻, PF₆⁻, SbF₆⁻, R⁵SO₃⁻, R^{5'} COO⁻, BF₄⁻, or BR⁶₄⁻, wherein R⁵, R^{5'} and R⁶ as defined above.

According to a particular embodiment of the invention, the stabilizing acid can be HCl, H₃PO₄, H₂SO₄ and R^{5'}COOH, such as MeCOOH or CF₃COOH.

According to a particular embodiment of the invention, the aldehyde derivatives of formula (I) are those wherein:
Q represents a CH₂COOH or COOH group, or a group of formula 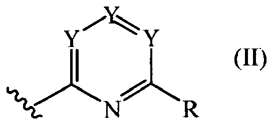 in which R represents a hydrogen atom or a C₁₋₃ alkyl or alkyoxy group, a CHO or COOH group or a phenyl, furyl or thiofuryl group optionally substituted;
each Y, simultaneously or independently, represents a CR group, with R being defined as above, or a nitrogen atom, provided that two Y are CR groups; and two R groups, taken together, may form an unsaturated or aromatic cycle containing 5 or 6 atoms and including the carbon atoms to which said R is bound, said cycle being substituted or unsubstituted and optionally containing one or two additional nitrogen or oxygen atoms.

Examples of possible substituents of said R comprise one or two groups such as R¹, OH, OR¹, COOR⁴ or CHO, with R¹ representing a C₁-C₃ hydrocarbon group or a phenyl group, optionally substituted by the same groups as defined above, R⁴ representing a hydrogen atom or an alkali metal cation.

According to anyone of the above embodiments of formula (I), said R represents a hydrogen atom or a C₁₋₃ alkyl group, a CHO or COOH group or a phenyl, group optionally substituted.

According to anyone of the above embodiments of formula (I), said aldehyde derivative is ideally odorless or has only a weak odor. In particular said compound of formula (I) could be characterized by a vapor pressure of below about 0.05 Pa (as obtained by calculation using the software EPIwin v 3.10, available at the US Environmental Protection Agency, 2000) and represents a particularly appreciated example, especially for what concerns the use of the present invention in the perfumery industry.

According to a particular aspect of the invention, said aldehyde derivative is a compound of formula (III), (III') or (III") 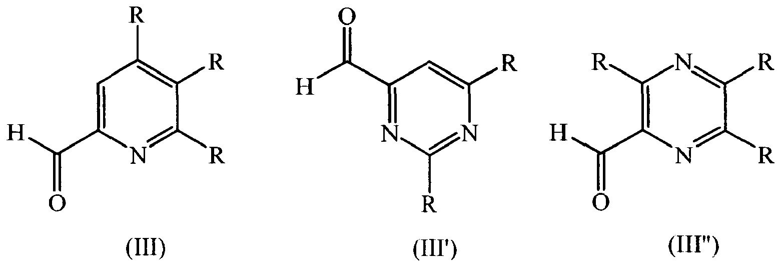 wherein R has the meaning defined above.

More specifically, as non-limiting examples of aldehyde derivatives of formula (I) described in the above-mentioned embodiments, one may cite the following:
i) pyridine-2-carboxaldehyde, methyl 4-(6-formylpyridin-2-yl)benzoate, 6-(1,3-benzodioxol-5-yl)pyridine-2-carboxaldehyde, 4-(6-formylpyridin-2-yl)benzonitrile, 6-(4-(methylsulfonyl)phenyl)pyridine-2-carboxaldehyde, 6-(4-methylthio)phenyl)pyridine-2-carboxaldehyde, 6-(2- or 3-thienyl)pyridine-2-carboxaldehyde, quinoline-2-carboxaldehyde or pyridine-2,6-dicarboxaldehyde;
ii) pyrimidine-4-carboxaldehyde, pyrimidine-4,6-dicarboxaldehyde, 2-phenylpyrimidine-4,6-dicarboxaldehyde or pyrimidine-2,4,6-tricarboxaldehyde;
iii) pyrazine-2-carboxaldehyde, quinoxaline-2-carboxaldehyde or pyrazine-2,3- or 2,5- or 2,6-dicarboxaldehyde;
iv) oxoacetic acid;
with all these derivatives of formula (I) being optionally substituted on the phenyl group (if present) by one to three methyl, methoxy, hydroxy or carboxylate groups.

According to some specific embodiments, pyridine-2-carboxaldehyde, pyridine-2,6-dicarboxaldehyde, pyrimidine-4-carboxaldehyde, pyrimidine-4,6-dicarboxaldehyde, 2-phenylpyrimidine-4,6-dicarboxaldehyde, pyrazine-2-carboxaldehyde, pyrazine-2,5-dicarboxaldehyde or oxoacetic acid are particularly suitable derivatives of formula (I).

The composition, may comprise at least two types of compounds (aldehyde (I) and optionally the stabilizing salt). The molar ratio between the total amount of the aldehyde derivative(s) according to formula (I) and the total amount of stabilizing salt(s) can be comprised between 1:0 and 4:1 or even between 1:2 and 4:1, preferably between 0.9:1 and 3:1, even more preferably at about 2:1.

In all the aspects of the above-described invention, active compounds, and in particular the perfuming ones, are mentioned. Said active ingredients are another important element of the dynamic mixture according to the present invention.

Examples of perfuming alcohols are available in perfumery handbooks or in the specialized literature or in the art patents, as mentioned further below.

Said perfuming compounds comprise, preferably, between 5 or 6 and 15 carbon atoms.

According to an embodiment of the invention, said perfuming alcohol has a molecular weight comprised between 90 and 210 g/mol and can be advantageously selected from the group consisting of primary, secondary or tertiary alcohols of formula R⁷OH, wherein R⁷ represents a C_{4 or 5 or 6}-C₁₅ saturated or unsaturated, linear or branched, mono- or bicyclic hydrocarbon group, optionally substituted with one, two or three C₁-C₃ alkyl or alkenyl, methoxy, ethoxy groups and/or phenyl groups optionally substituted with one, two or three C₁-C₃ alkyl or alkenyl, methoxy, ethoxy groups.

Although it is impossible to provide an exhaustive list of known alcohols of the formula R⁷OH which may be used according to the invention one might cite the following alcohols as non-limiting examples: 4-allyl-2-methoxyphenol (eugenol), 3-benzyl-3-pentanol, butanol, 4-cyclohexyl-2-methylbutan-2-ol (origin: Firmenich SA, Geneva, Switzerland), 2-cyclohexylpropanol, decanol, 9-decenol (Rosalva, origin: International Flavors and Fragrances, New York, USA), (2,4-dimethylcyclohex-3-enyl)methanol, (2,4-dimethylcyclohexyl)methanol, 2-(1,1-dimethylethyl)-4-methylcyclohexanol, 2,6-dimethytheptan-2-ol. 3,7-dimethyl-7-hydroxyoctanal, 3,7-diniethyl-1,6-nonadien-3-ol, 6,8-dimethylnonan-2-ol, 4,8-dimethyl-7-nonen-2-ol, (E)-3,7-dimethyl-2,6-octadienol (geraniol), (Z)-3,7-dimethyl-2,6-octadienol (nerol), 3,7-dimethyl-3,6-octadienol, 3,7-dimethyl-1,6-octadien-3-ol (linalool), 3,7-dimethyloctane-1,7-diol (hydroxycitronellol), 3,7-dimethyloctanol, 2,6-dimethyloctan-2-ol (tetrahydromyrcenol), 3,7-dimethyloctan-3-ol, 3,7-dimethyloct-6-enol (citronellol), 3,7-dimethyloct-7-enol, 2,6-dimethyloct-7-en-2-ol (dihydromyrcenol), (E)-3,3-dimethyl-5-(2,2,3-trimethyl-3-cyclopenten-1-yl)-4-penten-2-ol (Polysantol^{®}, origin: Firmenich SA, Geneva, Switzerland), dodecanol, 1,8-epoxy-p-menthane (eucalyptol), 2-ethyl-1-hexanol, ethyl 3-hydroxy hexanoate, 6-ethyl-3-methyl-5-octenol, 5-ethylnonan-2-ol. 2-ethyl-4-(2,2,3-trimethylcyclopent-3-enyl)but-2-enol, 1-heptanol, hexanol, hexan-2-ol, 3-hexenol, 4-hexenol, 3-hydroxybutan-2-one, 4-hydroxy-3-ethoxybenzaldehyde (ethylvanillin), 4-hydroxy-3-methoxybenzaldehyde (vanillin), 4-(4-hydroxy-3-methoxyphenyl)butan-2-one, 2-(hydroxymethyl)nonan-2-one, 4-(4-hydroxy-1-phenyl)butan-2-one (raspberry ketone), 4-isopropylcyclohexanol, 1-(4-isopropyl-1-cyclohexyl)ethanol, (4-isopropyl-1-cyclohexyl)methanol, (4-isopropylphenyl)methanol, 7-p-menthanol (Mayol^{®}, origin: Firmenich SA, Geneva, Switzerland), p-menthan-3-ol, p-menthan-8-ol, p-menthen-4-ol, p-menthen-8-ol, p-menth-8-enol, p-menth-8-en-2-ol, p-menth-8-en-3-ol. 7-methoxy-3,7-dimethyloctan-2-ol, 2-methoxyphenol, 2-methoxy-2-phenylethanol, (4-methoxyphenyl)methanol (anisyl alcohol), 2-methoxy-4-(1-propenyl)phenol (isoeugenol), 2-methoxy-4-propyl-1-cyclohexanol (Tarragol^{®}, origin: Firmenich SA, Geneva, Switzerland), 2-methoxy-4-propylphenol, 3-(4-methylcyclohex-3-enyl)butanol, 4-methyl-3-decenol, 4-methyl-3-decen-5-ol (origin: Givaudan SA, Geneva, Switzerland), 4-(1-methylethyl)cyclohexylmethanol, 2-methyl-4-phenylbutan-2-ol, 3-methyl-4-phenylbutan-2-ol, 1-(4-methylphenyl)ethanol, 2-(2-methylphenyl)ethanol, 2-methyl-4-phenylpentanol, 2-methyl-5-phenylpentanol, 3-methyl-5-phenylpentanol (phenylhexanol, origin: Firmenich SA, Geneva, Switzerland), 4-methyl-1-phenylpentan-2-ol, 2-methyl-1-phenylpropan-2-ol, 2-(4-methylphenyl)propan-2-ol, 3-methyl-5-(2,2,3-trimethyl-3-cyclopenten-1-yl)-4-penten-2-ol (Ebanol^{®}, origin: Givaudan SA, Geneva, Switzerland), 2-(2-methylpropyl)-4-hydroxy-4-methyl-tetrahydropyrane, 2-methyl-4-(2,3,3-trimethyl-2-cyclopenten-1-yl)-2-butenol (Santaliff^{®}, origin: International Flavors and Fragrances, New York, USA), 3-methyl-5-(2,2,3-trimethylcyclopentyl-3-enyl)pent-4-en-2-ol, 2,6-nonadienol, 1-nonanol, 6-nonenol, 1,2,3,4,4a,5,8,8a-octahydro-2,2,6,8-tetramethyl-1-naphthalenol, octanol, octan-2-ol, octan-3-ol, 1-octen-3-ol, 3,4,5,6,6-pentamethylheptan-2-ol (Kohinool^{®}, origin: International Flavors and Fragrances, New York, USA), 2-pentyl-1-cyclopentanol, perhydro-4,8a-dimethyl-4a-naphthalenol, 2-phenoxyethanol, 4-phenylbutan-2-ol, 4-phenyl-3-buten-2-ol, 1-phenylethanol, 2-phenylethanol, 1-phenylhexan-2-ol, 1-phenylpentan-2-ol, 2-phenylpropanol, 2-phenylpropanol, 3-phenylpropanol, 1-phenylpropan-2-ol, 3-phenyl-2-propenol, 2-tert-butylcyclohexanol (Verdol, origin International Flavors and Fragrances, New York, USA), 4-tert-butylcyclohexanol, 1-(2-tert-butyl-cyclohexyloxy)butan-2-ol, 2-tert-butyl-4-methyl-1-cyclohexanol, tetrahydro-2-isobutyl-4-methyl(2H)pyran-4-ol (Horol^{®}, origin: Firmenich SA, Geneva, Switzerland), 2-(tetrahydro-5-methyl-5-vinyl-2-furyl)propan-2-ol, 1-(2,2,3,6-tetramethylcyclohex-1-yl)hexan-3-ol (Limbanol^{®}, origin: Firmenich SA, Geneva, Switzerland), 2,6,10,10-tetramethyl-1-oxaspiro[4.5]decan-6-ol, 2,6,6,8-tetramethyltricyclo[5.3.1.0(1,5)]undecan-8-ol (cedrenol), (+)-(1R,2R)-1,3,3-trimethylbicyclo[2.2.1]heptan-2endo-ol (fenchol), (+)-(1R,2S)-1,7,7-trimethylbicyclo[2.2.1]heptan-2-ol (borneol), 2,6,6-trimethylbicyclo[3.1.1]heptan-3-ol, 3-(5,5,6-trimethylbicyclo[2.2.1]hept-2-yl)cyclohexanol (Sandela^{®}, origin: Givaudan SA, Geneva, Switzerland), 4-(5,5,6-trimethylbicyclo[2.2.1]hept-2-yl)cyclohexanol, 3,3,5-trimethylcyclohexanol, 4-(2,6,6-trimethyl-2-cyclohexen-1-yl)butan-2-ol, 4-(2,6,6-trimethyl-1-cyclohexen-1-yl)-3-buten-2-ol (beta-ionol), (E)-4-(2,6,6-trimethyl-2-cyclohexen-1-yl)-3-buten-2-ol (alpha-ionol), (2,4,6-trimethylcyclohex-3-enyl)methanol, 1-(2,2,6-trimethyl-1-cyclohexyl)hexan-3-ol, 5-(2,2,3-trimethyl-3-cyclopentenyl)-3-methylpentan-2-ol, 3,7,11-trimethyl-2,6,10-dodecatrienol (farnesol), 3,7,11-trimethyl-1,6,10-dodecatrien-3-ol (nerolidol), 3,3,5-trimethythexanol, undecanol, undecan-2-ol and 10-undecenol;
wherein primary and secondary alcohols represent, in an embodiment of the invention, particularly useful fragrance alcohols, with primary alcohols being particularly preferred.

Furthermore, according to any of the embodiments mentioned above, said perfuming alcohol is advantageously characterized by a vapor pressure above 2.0 Pa, as obtained by calculation using the software EPIwin v 3.10 (available at the US Environmental Protection Agency, 2000). According to another embodiment, said vapor pressure is above 5.0, or even above 7.0 Pa.

As mentioned further above, all these embodiments apply also in the case of the active ingredient being a flavoring, malodor counteracting, pharmaceutical, bactericide, fungicide, insect repellent or attractant ingredient.

The dynamic mixture can be obtained by reacting a composition, comprising at least one specific aldehyde derivative and, optionally, a stabilizing salt, with one or more perfuming ingredients in a medium. Said medium could be the active volatile alcohol or alcohols or a perfume oil (a mixture of said active volatile alcohol and other perfuming co-ingredients), optionally comprising a solvent of current use in the perfumery art. A detailed description of the nature and type of solvents commonly used in perfumery cannot be exhaustive. However, one can cite as non-limiting examples solvents such as dipropyleneglycol, diethyl phthalate, isopropyl myristate, benzyl benzoate, 2-(2-ethoxyethoxy)-1-ethanol or ethyl citrate, which are the ones most commonly used. Other solvents also used in perfumery can be acetone, ethanol, water, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company).

According to a particular embodiment of the invention said solvents are non-hydroxylic solvents, or in case of mixtures of solvents contains less than 35% molar amount of hydroxylic solvents, or even less than 10% or less than 5%.

The invention's dynamic mixture can be obtained by admixing together with the composition, comprising at least one specific aldehyde derivative and, optionally, a stabilizing salt, at least one perfuming alcohol. Although this fact is very useful in the perfumery art, where compounded perfumery ingredients are used frequently to achieve more pleasant and natural scents, it was definitively not evident that this could be operational. Indeed, the presence of several compounds capable of reacting all together (each of them with different stabilities and reactivities), could have easily led to a negative impact of the release of the individual active volatile alcohol thus resulting in a negative hedonic effect. This is not the case with the present invention. Therefore, a dynamic mixture obtained by reacting together the composition with at least two, or even at least three perfuming compounds is particularly appreciated.

As mentioned above, the invention's dynamic mixture comprises several starting components that may react, in a reversible manner, between them to form addition products.

Now, a further aspect of the present invention concerns the dynamic mixtures themselves. Indeed, the above-mentioned dynamic mixtures are also new, and therefore represent another object of the invention. So another aspect of the present invention are the dynamic mixtures for the controlled release of active volatile alcohols. In particular we can mention dynamic mixtures wherein the active volatile alcohol is a perfuming one, as described above.

A further aspect of the present invention concerns the compositions themselves, which are also new.

It is believed that the main components of the dynamic mixture are the free active volatile alcohol, the components of the composition, comprising at least one specific aldehyde derivative and, optionally, a stabilizing salt, and the resulting addition products (such as the corresponding hemiacetal derivatives). A specific example of such a mixture and equilibrium is presented in Scheme (I):
Scheme (I) : Example showing some of the species in equilibrium and believed to be formed in a dynamic mixture obtained from one alcohol and one specific aldehyde derivative and a stabilizing salt.

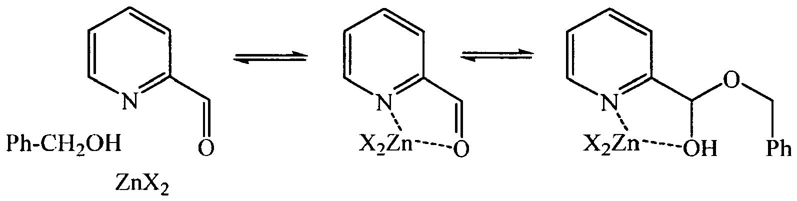

The stability of the hemiacetal may be modulated by protonation or via metal complex formation with the stabilizing salt. According to the structure of the specific aldehydes, monomeric, dimeric or even trimeric complexes can be formed.

Due to its nature, the invention's dynamic mixture circumvents the problem of product instability observed with prior art precursors, by the fact that a dynamic equilibrium is spontaneously set up between these compounds. This instability problem is avoided in a way significantly different from the one generally described in the prior art where it is always mentioned that it is preferable to isolate a profragrance. In the case of the present invention, the equilibrium is stable during product storage as long as the consumer product parameters (such as concentration, temperature, pH or humidity, the presence of surfactant etc.) are kept constant. At a given set of parameters, the time required to reach the equilibrium state mainly depends on the kinetic rate constant of the slowest step involved in the formation of the products of the equilibrium.

The invention's dynamic mixture is furthermore able to stabilize active volatile alcohols, against degradation such as oxidations, by reversibly forming an addition product between a compound of formula (I), a stabilizing salt and the active volatile alcohol and thus reversibly protect the hydroxyl function as an hemiacetal function. The spontaneous reversible formation of a significant amount of hemiacetals in the dynamic mixture is thus expected to stabilize the alcohol functionality of the active volatile alcohol to a large extent.

As mentioned above, the dynamic mixture of the invention comprises various components. It is believed that, once the dynamic mixture is generated, the free volatile active alcohol starts to evaporate, diffusing in the surrounding environment their typical scent. Said evaporation perturbs the chemical equilibrium and the various addition products start to decompose so as to restore the equilibrium. The consequence of such reequilibration is the regeneration of the free volatile active alcohol, thus maintaining its concentration relatively constant over time and avoiding a too rapid evaporation.

Now, it has been observed that the various physical or thermodynamic properties of the dynamic mixture, e.g. the amount of addition products formed, can be influenced by the chemical nature of the perfuming compounds or of the derivatives of formula (I) as well as of the stabilizing salt. Another way to influence the above-mentioned properties is to modify the molar ratio between said perfuming compounds and the composition, as well as the molar ratio between said derivative (I) and salt. For instance, the lower the molar ratio between perfuming compounds and the composition, the longer takes the evaporation of all the perfuming compounds. The presence of other ingredients (such as surfactants, emulsifiers, gelators or others) typically used in the final consumer product formulation may also influence the above-mentioned properties.

Therefore, by varying the chemical structure of the mixture's constituents and their ratio, it is possible to fine-tune the release properties of the invention's dynamic mixture, so as to adapt its behavior to the specific requirement of the targeted consumer product.

According to the final application, a broad range for the speed of evaporation of the perfuming compound may be desirable.

The ratio between the total molar amount of perfuming alcohol and the total molar amount of the aldehyde derivative of formula (I) can be comprised between 1:2 and 100:1, preferably between 1:1 and 50:1, even more preferably between 1:1 and 10:1.

The amount of free perfuming alcohol present in the equilibrated dynamic mixture is comprised between 1 and 97%, preferably between 5 and 95% or even more preferably between 25 and 90%.

Another advantage of the invention resides in the fact that it is possible to fine-tune the thermodynamic behavior of the dynamic mixture by selecting the nature of the salt or of the aldehyde (I). It is therefore conceivable to design dynamic mixtures comprising, for instance, a derivative of formula (I) and a stabilizing salt which allows a fast release of a specific perfuming alcohol (which will be perceivable at the beginning of the consumer use only) and a second derivative of formula (I) and a stabilizing salt which allows a release of the same specific perfuming alcohol, or of another, a very slow release (which will be perceivable even after an important delay from the direct consumer use).

Moreover, another object of the present invention concerns also a composition comprising the invention's dynamic mixture. This concerns also in particular a perfuming composition comprising:
i) as perfuming ingredient, a dynamic mixture as defined above;
ii) at least one ingredient selected from the group consisting of a perfumery carrier and a perfumery base; and
iii) optionally at least one perfumery adjuvant.

Preferably, in said perfuming composition the perfumery carrier, perfumery base and perfumery adjuvant have a total molar amount of alcohols which is equal to or lower than the molar amount of aldehyde derivatives of formula (I) of the dynamic mixture.

By "perfumery carrier" we mean here a material which is practically neutral from a perfumery point of view, i.e. which does not significantly alter the organoleptic properties of perfuming ingredients. Said carrier may be a liquid. As liquid carrier one may cite, as non-limiting examples, an emulsifying system, i.e. a solvent and a surfactant system, or a solvent commonly used in perfumery as defined above.

By "perfumery base" we mean here a composition comprising at least one perfuming co-ingredient. Said perfuming co-ingredient is not an aldehyde or ketone as defined above for the dynamic mixture. Moreover, by "perfuming co-ingredient" it is meant here a compound, which is used in perfuming preparation or composition to impart a hedonic effect. In other words such a co-ingredient, to be considered as being a perfuming one, must be recognized by a person skilled in the art as being able to impart or modify in a positive or pleasant way the odor of a composition, and not just as having an odor.

The nature and type of the perfuming co-ingredients present in the base do not warrant a more detailed description here, which in any case would not be exhaustive, the skilled person being able to select them on the basis of its general knowledge and according to intended use or application and the desired organoleptic effect. In general terms, these perfuming co-ingredients belong to chemical classes as varied as alcohols, aldehydes, ketones, esters, lactones, ethers, acetates, nitriles, terpene hydrocarbons, nitrogenous or sulphurous heterocyclic compounds and essential oils, and said perfuming co-ingredients can be of natural or synthetic origin. A further class of perfuming co-ingredients can be the alcohols which are not part of the stabilized hemiacetal present in the dynamic mixture.

Many of these co-ingredients are in any case listed in reference texts such as the book by S. Arctander, Perfume and Flavor Chemicals, Montclair, New Jersey, USA (1969), or its more recent versions, or in other works of a similar nature, as well as in the abundant patent literature in the field of perfumery. It is also understood that said co-ingredients may also be compounds known to release in a controlled manner various types of perfuming compounds.

By "perfumery adjuvant" we mean here an ingredient capable of imparting additional added benefit such as a color, a particular light resistance, chemical stability (e.g. antioxidants) and others. A detailed description of the nature and type of adjuvant commonly used in perfuming bases cannot be exhaustive, but it has to be mentioned that said ingredients are well known to a person skilled in the art.

An invention's composition consisting of an invention's dynamic mixture and at least one perfumery carrier represents a particular embodiment of the invention as well as a perfuming composition comprising an invention's dynamic mixture, at least one perfumery carrier, at least one perfumery base, and optionally at least one perfumery adjuvant.

As anticipated above, the invention's dynamic mixtures or compositions can be advantageously used for bringing a benefit to consumer products, such as its perfuming. Indeed, said mixture possesses several other properties that make it particularly suitable for this purpose. Consequently, a consumer article comprising the invention's dynamic mixture is also an object of the present invention.

All the above-mentioned properties, i.e. improved substantivity, prolonged time of evaporation (long-lastingness), improved stability over aggressive agents, and improved substantivity, are very important for a perfuming composition. Indeed, when said compositions are intended for use in fine perfumery, the invention's mixture may allow the creation of new perfuming effects which are otherwise difficult to be achieved, such as a fresh green note being present over several hours. In the case of perfuming compositions intended for the functional perfumery, the above-mentioned properties are also very important. For example, perfuming ingredients present as such in washing compositions, or air fresheners, which have generally little substaintivity on a surface are consequently often eliminated very quickly. This problem can be solved by using the invention's dynamic mixture, which possesses an improved stability over storage and substantivity.

Therefore, the mixtures according to the invention, owing to a lower and more uniform evaporation per unit of time, resulting in a controlled release of odoriferous molecules, can be incorporated in any application requiring the effect of prolonged liberation of an odoriferous component as defined hereinabove and furthermore can improve the fragrance and a freshness imparted.

Consequently, the invention concerns also a consumer article comprising:
i) as active ingredient, a dynamic mixture as defined above; and
ii) a consumer product base;
is also an object of the present invention.

In particular, said consumer article can be a perfumed article comprising:
i) as perfuming ingredient, a dynamic mixture as defined above; and
ii) a consumer product base.

Preferably, in perfumed articles the consumer product base has a total molar amount of alcohols which is equal to or higher than the molar amount of derivatives of formula (I) of the dynamic mixture.

For the sake of clarity, it has to be mentioned that, by "consumer product base" we mean here a consumer product which is compatible with a perfume or perfuming ingredients and which can be liquid or solid, such as a suspension, an emulsion, a gel or a paste or a cream. In other words, a perfumed article according to the invention comprises the functional formulation, as well as optionally additional benefit agents, corresponding to a consumer product, e.g. a conditioner, a softener or an air freshener, and an olfactively effective amount of an invention's dynamic mixture.

The nature and type of the constituents of the liquid consumer product base do not warrant a more detailed description here, which in any case would not be exhaustive, the skilled person being able to select them on the basis of its general knowledge and according to the nature and the desired effect of said article.

Example of suitable consumer products can be solid or liquid detergents and fabric softeners as well as hygiene products, body-care products or cosmetics. In particular said consumer product can be a perfume, cologne or after-shave lotion, a perfumed soap, shower or bath mousse, oil or gel, or a hair care product such as a shampoo or a hair spray, a deodorant or antiperspirant, an air freshener or a "ready to use" powdered air freshener, or a detergent. As "detergents" are intended consumer products bases such as detergent compositions or cleaning products for washing up or for cleaning various surfaces, e.g. intended for textile, dish or hard-surface treatment, whether they are intended for domestic or industrial use. Other perfumed articles are fabric refreshers, ironing waters, papers, wipes or bleaches.

Typical examples of fabric detergents or softener compositions into which the compounds of the invention can be incorporated are described in Ullman's Encyclopedia of Industrial Chemistry, vol. A8, pages 315-448 (1987) and vol. A25, pages 747-817 (1994); Flick, Advanced Cleaning Product Formulations, Noye Publication, Park Ridge, New Jersey (1989); Showell, in Surfactant Science Series, vol. 71: Powdered Detergents, Marcel Dekker, New York (1988); Proceedings of the World Conference on Detergents (4th, 1998, Montreux, Switzerland), AOCS print.

Preferred consumer products are perfumes, air fresheners, deodorants or antiperspirants, cosmetic preparations, ironing waters, softener bases, liquid or solid detergents, fabric refreshers, shampoos or hair sprays.

Even more preferred consumer products are perfumes, softener bases or fabric refreshers, liquid or solid based deodorants or antiperspirants, liquid or solid detergents, or air fresheners.

Some of the above-mentioned articles may represent an aggressive medium for the invention's compounds, so that it may be necessary to protect the latter from premature decomposition, for example by encapsulation.

The proportions in which the dynamic mixture according to the invention can be incorporated into the various aforementioned articles or compositions vary within a wide range of values. These values are dependent on the nature of the article or product to be perfumed and on the desired olfactory effect as well as the nature of the co-ingredients in a given composition when the dynamic mixtures according to the invention are mixed with perfuming co-ingredients, solvents or additives commonly used in the art.

Despite the fact that the dynamic mixtures containing stabilized hemiacetals are usually prepared in solution, they may well serve, after removal of the solvent, as ingredients in solid product formulations, such as deodorants or antiperspirants and detergents, e.g. detergent powders, or soaps. Indeed, once said solid product are in contact with a liquid the various chemical equilibrium take place again.

For example, typical concentrations are in the order of 0.1 % to 50 % by weight, or even more, of the invention's dynamic mixture based on the weight of the composition into which they are incorporated. Concentrations lower than these, such as in the order of 0.01% to 10% by weight, can be used when these dynamic mixtures are applied directly in the perfuming of the various consumer products mentioned hereinabove.

In particular, said consumer article can be a flavored article comprising:
i) as flavoring ingredient, a dynamic mixture as defined above; and
ii) a foodstuff base.

For the sake of clarity, it has to be mentioned that, by "foodstuff base", we mean here an edible product, e.g. a food or a beverage. Therefore, a flavored article according to the invention comprises the functional formulation, as well as optionally additional benefit agents, corresponding to a desired edible product, e.g. foods or beverages, and a flavor effective amount of at least an invention's compound.

The nature and type of the constituents of the foodstuffs or beverages do not warrant a more detailed description here, which in any case would not be exhaustive, the skilled person being able to select them on the basis of its general knowledge and according to the nature of said product.

Non-limiting examples of suitable foodstuff bases include a bakery, a dairy, a confectionary, a savory, an infusion, a soft drink, a flavored water and a juice product. In particular one may cite foodstuff bases such as a chewing gum, a yogurt, milk, a hot or cold tea drink, a carbonated or non-carbonated soft drinks, an instant drink, a soup, a fruit juice.

The proportions in which the compounds according to the invention can be incorporated into the various aforementioned articles or compositions vary within a wide range of values. These values are dependent on the nature of the article to be flavored and on the desired organoleptic effect as well as the nature of the co-ingredients in a given base when the compounds according to the invention are mixed with flavoring co-ingredients, solvents or additives commonly used in the art.

For example, typical concentrations are in the order of 0.01 % to 1% by weight, can be used when these dynamic mixtures are applied directly in the perfuming of the various consumer products mentioned hereinabove.

Another object of the present invention relates to a method for the perfuming of a surface characterized in that said surface is treated in the presence of a dynamic mixture as defined above. Suitable surfaces are, in particular, textiles, hard surfaces, hair and skin.

Moreover, an additional aspect of the present invention is a method for prolonging the perfuming effect of a perfuming alcohol, as defined above, characterized in that a composition, comprising at least one specific aldehyde derivative and optionally a stabilizing salt as defined above, is added to a perfuming composition or perfumed article containing at least one of said alcohols. In other words, it concerns the use of a derivative of a controlling composition, as defined above, as additive to prolong the perfuming effect of a perfuming compositions or perfumed article containing at least one perfuming compound as defined above.

### Examples

The invention will now be described in further detail by way of the following examples, wherein the abbreviations have the usual meaning in the art, temperatures are indicated in degrees centigrade (°C). If not stated otherwise, the NMR spectral data were recorded on Bruker Avance or AMX 400 spectrometers at 400 MHz for ¹H and at 100.6 MHz for ¹³C, the chemical displacements δ are indicated in ppm with respect to TMS as the standard, the coupling constants J are expressed in Hz. Commercially available reagents and solvents were used without further purification if not stated otherwise. Citronellol used for the measurements was (R)-citronellol.

### Use of active alcohols

The following examples illustrate the formation of dynamic mixtures using perfuming or flavoring ingredients as active alcohols. However, they are also representative for the generation of dynamic mixtures according to the present invention in which the active alcohols are useful as insect repellants or attractants. Some of the compounds described in the following examples, such as butanol, citronellol, geraniol, (Z)-3-hexenol or 2-phenylethanol, are also known to be insect attractants or repellents (see for example: A. M. El-Sayed, The Pherobase 2005, http://www.pherobase.net) and/or to be active against bacteria (see for example: WO 01/24769 or EP 1 043 968).

### Example 1

### Formation of dynamic mixtures of stabilized hemiacetals in the absence of a stabilizing salt

Mixtures of an active alcohol (butanol) and an aldehyde (pyridine-2-carboxaldehyde (**1**), pyridine-2,6-dicarboxaldehyde (**2**) or 2-phenylpyrimidine-4,6-dicarboxaldehyde (**3**)) in CDCl₃ were prepared at different stoichiometric ratios while keeping the final aldehyde concentration at 0.15 mol/L. The mixtures were then analyzed by NMR spectroscopy. The following compositions were obtained:

| Aldehyde | Equivalents of alcohol | Amount of remaining aldehyde [%] | Amount of mono-hemiacetal fomied [%] | Amount of bis-hemiacetal formed [%] |
|---|---|---|---|---|
| **2** | | 94 | 6 | 0 |
| **3** | | 83 | 17 | 0 |
| **1** | 1.0 eq. | 97 | 3 | --- |
| **2** | | 87 | 13 | 0 |
| **3** | | 68 | 32 | 0 |
| **1** | 3.0 eq. | 94 | 6 | --- |
| **2** | | 70 | 30 | 0 |
| **3** | | 30 | 56 | 14 |
| **1** | 6.0 eq. | 89 | 11 | --- |
| **2** | | 56 | 43 | 1 |
| **3** | | 17 | 56 | 27 |
| | | | | |

The following compositions were obtained when using geraniol (**A**) or citronellol (**B**) instead of butanol as the active alcohol:

| Aldehyde | Equivalents of alcohol | Amount of remaining alcohol [%] | Amount of mono-hemiacetal formed [%] | Amount of bis-hemiacetal formed [%] |
|---|---|---|---|---|
| **1** | **A**: 1.0 eq. | 94 | 6 | --- |
| | **B**: 1.0 eq. | 97 | 3 | --- |
| **2** | **A**: 2.0 eq. | 74 | 26 | 0 |
| | **B**: 2.0 eq. | 78 | 22 | 0 |

### Example 2

### Formation of dynamic mixtures of stabilized hemiacetals using a protic acid as the stabilizing salt

Mixtures of an active alcohol (butanol, (**A**), 2-methoxyethanol (**B**), isopropanol (**C**), citronellol (**D**), geraniol (**E**)) and an aldehyde (pyridine-2-carboxaldehyde (**1**), pyridine-2,6-dicarboxaldehyde (**2**) or 2-phenylpyrimidine-4,6-dicarboxaldehyde (**3**)) and 3 molar equivalents of deuterated trifluoroacetic acid (with respect to the aldehyde) in CDCl₃ were prepared at different stoichiometric ratios while keeping the final aldehyde concentration at 0.15 mol/L. The mixtures were then analyzed by NMR spectroscopy. The following compositions were obtained:

| Aldehyde | Equivalents of alcohol | Amount of remaining aldehyde or alcohol [%] | Amount of mono-hemiacetal formed [%] | Amount of bis-hemiacetal formed [%] |
|---|---|---|---|---|
| **1** | **A**: 1.0 eq. | 32 | 68 | --- |
| | **A**: 3.0 eq. | 14 | 86 | --- |
| | **B**: 1.0 eq. | 41 | 59 | --- |
| | **B**: 3.0 eq. | 18 | 82 | --- |
| | **C**: 1.0 eq. | 55 | 45 | --- |
| | **C**: 3.0 eq. | 25 | 75 | --- |
| | **D**: 1.0 eq. | 75 | 25 | --- |
| | **E**: 1.0 eq. | 43 | 57 | --- |
| **2** | **A**: 2.0 eq. | 72 | 6 | 22 |
| | **A**: 6.0 eq. | 48 | 5 | 47 |
| | **B**: 2.0 eq. | 84 | 5 | 11 |
| | **B**: 6.0 eq. | 69 | 9 | 22 |
| **3** | **A**: 2.0 eq. | 70 | 30 | 0 |
| | **B**: 2.0 eq. | 60 | 40 | 0 |

The following compositions were obtained in CD₃CN:

| Aldehyde | Equivalents of alcohol | Amount of remaining alcohol [%] | Amount of mono-hemiacetal formed [%] | Amount of bis-hemiacetal formed [%] |
|---|---|---|---|---|
| **1** | **D**: 1.0 eq. | 44 | 56 | --- |
| | **E**: 1.0 eq. | 42 | 58 | --- |

### Example 3

### Formation of dynamic mixtures of stabilized hemiacetals using a metal cation as the stabilizing salt

Mixtures of an active alcohol (butanol, (**A**), 2-methoxyethanol (**B**), isopropanol (**C**), citronellol (**D**), geraniol (**E**), 2-phenylethanol (**F**), (Z)-3-hexenol (**G**) or tert-butanol (**H**)) and an aldehyde (pyridine-2-carboxaldehyde (**1**), pyridine-2,6-dicarboxaldehyde (**2**) or oxoacetic acid (**4**)) and 0.5 molar equivalents of zinc (II) triflate (with respect to the aldehyde) in CD₃CN were prepared at different stoichiometric ratios while keeping the final aldehyde concentration at 0.15 mol/L. The mixtures were then analyzed by NMR spectroscopy. The following compositions were obtained:

| Aldehyde | Equivalents of alcohol | Amount of remaining aldehyde or alcohol [%] | Amount of mono-hemiacetal formed [%] | Amount of bis-hemiacetal formed [%] |
|---|---|---|---|---|
| **1** | **A**: 1.0 eq. | 59 | 41 | --- |
| | **B**: 1.0 eq. | 67 | 33 | --- |
| | **C**: 1.0 eq. | 69 | 31 | --- |

| | | | | |
|---|---|---|---|---|
| | **D**: 1.0 eq. | 61 | 39 | --- |
| | **E**: 1.0 eq. | 60 | 40 | --- |
| | **F**: 1.0 eq. | 49 | 51 | --- |
| | **G**: 1.0 eq. | 64 | 36 | --- |
| **2** | **A**: 2.0 eq. | 19 | 0 | 81 |
| | **A**: 6.0 eq. | 5 | 0 | 95 |
| | **B**: 2.0 eq. | 30 | 0 | 70 |
| | **B**: 6.0 eq. | 19 | 0 | 81 |
| | **C**: 2.0 eq. | 30 | 0 | 70 |
| | **D**: 2.0 eq. | 23 | 77 | 0 |
| | **H**: 2.0 eq. | 57 | 0 | 43 |
| **4** | **D**: 1.0 eq. | 34 | 66 | --- |

The following compositions were obtained when using 0.5 equivalents of zinc chloride (with respect to the aldehyde) instead of zinc triflate as the stabilizing salt:

| Aldehyde | Equivalents of alcohol | Amount of remaining alcohol [%] | Amount of mono-hemiacetal formed [%] | Amount of bis-hemiacetal formed [%] |
|---|---|---|---|---|
| **1** | **D**: 1.0 eq. | 80 | 20 | --- |
| | **E**: 1.0 eq. | 79 | 21 | --- |
| | **F**: 1.0 eq. | 75 | 25 | --- |
| | **G**: 1.0 eq. | 82 | 18 | --- |

The following compositions were obtained when using zinc triflate as the stabilizing salt and a mixture of CD₃CN and D₂O (2: 1) instead of pure CD₃CN as the solvent:

| Aldehyde | Equivalents of alcohol | Amount of remaining alcohol [%] | Amount of mono-hemiacetal formed [%] | Amount of bis-hemiacetal formed [%] |
|---|---|---|---|---|
| **1** | **D**: 1.0 eq. | 97 | 3 | --- |
| | **E**: 1.0 eq. | 95 | 5 | --- |
| **2** | **E**: 2.0 eq. | 83 | 7 | 0 |

The following compositions were obtained when using zinc triflate as the stabilizing salt and acetone-D₆ instead of CD₃CN as the solvent:

| Aldehyde | Equivalents of alcohol | Amount of remaining alcohol [%] | Amount of mono-hemiacetal fomied [%] | Amount of bis-hemiacetal formed [%] |
|---|---|---|---|---|
| **1** | **D**: 1.0 eq. | 72 | 28 | --- |
| | **E**: 1.0 eq. | 60 | 40 | --- |
| | **F**: 1.0 eq. | 63 | 37 | --- |
| | **G**: 1.0 eq. | 66 | 34 | --- |

The following compositions were obtained when using copper (II) triflate as the stabilizing salt and acetone-D₆ as the solvent:

| Aldehyde | Equivalents of alcohol | Amount of remaining alcohol [%] | Amount of mono-hemiacetal formed [%] | Amount of bis-hemiacetal formed [%] |
|---|---|---|---|---|
| **1** | **E**: 1.0 eq. | ca. 85 | ca. 15 | --- |

### Example 4

### Formation of dynamic mixtures of stabilized hemiacetals using a stabilizing salt and several active alcohols

A mixture of pyridine-2-carboxaldehyde (1) and butanol (A), 2-methoxyethanol (B) and isopropanol (C) (3 equivalents each with respect to the aldehyde) in CD₃CN have been treated with 0.5 or 1 molar equivalent of zinc triflate (Zn²⁺) or of deuterated trifluoroacetic acid (D⁺) (with respect to the aldehyde), respectively, while keeping the final aldehyde concentration at 0.15 mol/L. The mixtures were then analyzed by NMR spectroscopy. The following compositions were obtained:

| Equivalents of stabilizing salt | Amount of remaining aldehyde [%] | Amount of hemiacetal formed with **A** [%] | Amount of hemiacetal formed with **B** [%] | Amount of hemiacetal formed with **C** [%] |
|---|---|---|---|---|
| Zn²⁺: 0.5 eq. | 5 | 46 | 21 | 28 |
| Zn ²⁺ : 1.0 eq. | 5 | 44 | 20 | 29 |
| D⁺: 0.5 eq. | 79 | 16 | 3 | 2 |
| D⁺: 1.0 eq. | 60 | 26 | 9 | 5 |

### Example 5

### Use of stabilized hemiacetals for the controlled release of active alcohols in a fabric softening application

A stabilized hemiacetal was prepared by mixing 0.6 mL of a solution containing 96.5 mg of pyridine-2-carboxaldehyde in 2 mL of CH₃CN. 0.6 mL of a solution containing 109.7 mg of 2-phenylethanol in 2 mL of CH₃CN and 0.6 mL of a solution containing 327.5 mg of zinc triflate as the stabilizing salt in 2 mL of CH₃CN, respectively. Similarly, a reference sample was prepared by adding 1.2 mL of CH₃CN to 0.6 mL of the 2-phenylethanol solution. Each of the samples was prepared in duplicate. The solutions were left standing at room temperature for 3 days. Then a series of four beakers, each contaning 1.8 g of a fabric softener formulation consisting of:

| | |
|---|---|
| Stepantex^{®} VL 90 A (origin: Stepan) | 16.5% (w/w) |
| Calcium chloride (10% aqueous solution) | 0.6% (w/w) |
| Demineralized water | 82.9% (w/w) |

was prepared. Then 1 mL of the stabilized hemiacetal solution was pipetted to into the first two beakers, respectively, and 1 mL of the 2-phenylethanol reference solution into each of the two other beakers. Then 600 mL of tap water and one cotton towel (EMPA cotton test cloth Nr. 221, origin: Eidgenössische Materialprüfanstalt (EMPA), pre-washed with an unperfumed detergent powder and cut to ca. 12 x 12 cm sheets) was added to each beaker. The sheets were agitated manually for 3 min, left standing for 2 min, then wrung out by hand, weighed to obtain a constant quantity of residual water and line-dried overnight. Two of the sheets (one with the dynamic mixture and one without) were analyzed the next day, the other two after 3 days. For the analysis, the sheets were each put into a headspace sampling cell (160 mL) thermostatted at 25°C and exposed to a constant air flow of 200 mL/min, respectively. The air was filtered through active charcoal and aspirated through a saturated solution of NaCl (to ensure a constant humidity of the air of ca. 75%). During 15 min the headspace system was left equilibrating, then the volatiles were adsorbed during 15 min on a clean Tenax^{®} cartridge, respectively. The sampling was repeated 7 times every 45 min. The cartridges were desorbed on a Perkin Elmer TurboMatrix ATD desorber coupled to a Carlo Erba MFC 500 gas chromatograph equipped with a J&W Scientific DB1 capillary column (30 m. i.d. 0.45 mm. film 0.42 µm) and a FID detector. The volatiles were analyzed using a two step temperature gradient starting from 70°C to 130°C at 3°C/min and then going to 260°C at 25°C/min. The injection temperature was at 240°C, the detector temperature at 260°C. Headspace concentrations (in ng/L) were obtained by external standard calibrations of the corresponding fragrance alcohol using ethanol solutions of five different concentrations. 0.1 µL of each calibration solution was injected onto Tenax^{®} cartridges, which were immediately desorbed under the same conditions as those resulting from the headspace sampling.

The following amounts of 2-phenylethanol were detected from the sample containing the dynamic mixture of stabilized hemiacetal as compared to the reference sample:

| | Dried for 1 day and analyzed after 210 min [ng/L] | Dried for 1 day and analyzed after 450 min [ng/L] | Dried for 3 days and analyzed after 210 min [ng/L] | Dried for 3 days and analyzed after 450 min [ng/L] |
|---|---|---|---|---|
| 2-Phenylethanol from stabilized hemiacetal | 106.4 | 120.8 | 115.0 | 122.7 |
| 2-Phenylethanol (Reference) | 103.5 | 124.3 | 37.3 | 69.3 |

The fact that comparable headspace concentrations were measured for the 2-phenylethanol released from the stabilized hemiacetal and the reference after 1 day, but much higher concentrations from the hemiacetal after 3 days clearly demonstrates the desired effect of long-lastingness (prolonged time of evaporation) obtained from the sample containing the stabilized hemiacetal.

The following headspace concentrations were measured on dry fabric after 3 days (using the thermodesorption and GC-instrumentation described in Example 6) when the experiment was carried out under the same conditions described above using 145.8 mg of FeCl₃ instead of 327.5 mg of zinc triflate (average values of two measurements):

| | Dried for 3 days and analyzed after 210 min [ng/L] | Dried for 3 days and analyzed after 450 min [ng/L] |
|---|---|---|
| 2-Phenylethanol from stabilized hemiacetal | 40.9 | 33.8 |
| 2-Phenylethanol (Reference) | 18.4 | 17.0 |

The desired long-lastingness was also observed using FeCl₃ as the stabilizing salt.

### Example 6

### Use of stabilized hemiacetals for the controlled release of active alcohols in a powder detergent application

A stabilized hemiacetal was prepared by mixing 1.2 mL of a solution containing 96.3 mg of pyridine-2-carboxaldehyde in 2 mL of CH₃CN, 1.2 mL of a solution containing 109.8 mg of 2-phenylethanol in 2 mL of CH₃CN and 1.2 mL of a solution of 327.4 mg of zinc triflate as the stabilizing salt in 2 mL of CH₃CN, respectively. Similarly, a reference sample was prepared by adding 2.4 mL of CH₃CN to 1.2 mL of the 2-phenylethanol solution. The solutions were left standing at room temperature for 3 days.

Then 1.8 g of a non-perfumed, commercially available powder detergent (Via Professional Sensitive, origin: Unilever), 2 mL of the stabilized hemiacetal solution (or the corresponding reference sample), 400 mL of tap water and two cotton sheets (see Example 5) were added to two stainless steel containers of a Linitest^{®} washing machine (origin: Heraeus), respectively. The containers were fixed inside the Linitest^{®} machine and rotated at 45°C for 20 min. The cotton sheets were rinsed by agitating them manually in 600 mL of tap water for 2 min, and line-dried overnight. Two of the sheets (one with the dynamic mixture and one without) were analyzed the next day, as described in Example 5. The volatiles were adsorbed during 30 min on a clean Tenax^{®} cartridge, respectively, and the sampling was repeated 7 times every 30 min. The cartridges were desorbed on a Perkin Elmer TurboMatrix 350 desorber coupled to a Perkin Elmer AutoSystem XL gas chromatograph equipped with a J&W Scientific DB1 capillary column (30 m, i.d. 0.45 mm, film 0.42 µm) and a Perkin Elmer TurboMass Upgrade MS detector. The volatiles were analyzed in the SIM mode using a two step temperature gradient starting from 60°C to 110°C at 2°C/min and then going to 260°C at 45°C/min. Headspace concentrations (in ng/L) were obtained by external standard calibrations as described in Example 5.

The following amounts of 2-phenylethanol were detected from the sample containing the dynamic mixture of stabilized hemiacetal as compared to the reference sample:

| | Dried for 1 day and analyzed after 105 min [ng/L] | Dried for 1 day and analyzed after 225 min [ng/L] | Dried for 1 day and analyzed after 345 min [ng/L] | Dried for 1 day and analyzed after 465 min [ng/L] |
|---|---|---|---|---|
| 2-Phenylethanol from stabilized hemiacetal | 13.8 | 13.5 | 12.6 | 12.9 |
| 2-Phenylethanol (Reference) | 13.6 | 10.9 | 9.6 | 8.1 |

Despite the fact that comparable headspace concentrations were measured at the start of the experiment, the amount of 2-phenylethanol released from the stabilized hemiacetal decreased more slowly than the amount obtained from the reference sample, thus demonstrating the desired effect of long-lastingness (prolonged time of evaporation) in the sample containing the stabilized hemiacetal.

### Example 7

### Use of stabilized hemiacetals for the controlled release of active alcohols in a deodorant application

A stabilized hemiacetal was prepared by mixing 0.6 mL of a solution containing 96.5 mg of pyridine-2-carboxaldehyde in 2 mL of CH₃CN, 0.6 mL of a solution containing 109.8 mg of 2-phenylethanol in 2 mL of CH₃CN and 0.6 mL of a solution containing 327.4 mg of zinc triflate as the stabilizing salt in 2 mL of CH₃CN, respectively. Similarly, a reference sample was prepared by adding 1.2 mL of CH₃CN to 0.6 mL of the 2-phenylethanol solution. The solutions were left standing at room temperature for 1 day. Then 5.0 g of a non-perfumed deodorant stick formulation with the following composition:

| | |
|---|---|
| Stearic acid | 5.05% (w/w) |
| 1,2-Propylene glycol | 57.02% (w/w) |
| Sodium hydroxide (20% in aqueous solution) | 4.24% (w/w) |
| Water | 30.30% (w/w) |
| Tetrasodium EDTA | 0.10% (w/w) |
| Ceteareth-25 | 1.52% (w/w) |
| PPG-3 myristyl ether | 1.52% (w/w) |
| Triclosan | 0.25% (w/w) |

was weighed in a glass vial and heated on a water bath to 80°C, before 1 mL of the stabilized hemiacetal (or 1 mL of the reference sample) was added. The vials were closed and the mixture was left cooling to room temperature to form a paste. After 24 h the vials were opened and a first measurement was performed. After standing for another day, a second measurement was carried out.

For the analysis, each vial (the one with the stabilized hemiacetal and the reference) was put into a headspace sampling cell (625 mL) and exposed to a constant air flow of 200 mL/min, respectively. The air was filtered through active charcoal and aspirated through a saturated solution of NaCl (to ensure a constant humidity of the air of ca. 75%). During 15 min the headspace system was left equilibrating, then the volatiles were adsorbed during 15 min on a clean Tenax^{®} cartridge, respectively. The cartridges were desorbed on a Perkin Elmer TurboMatrix 350 desorber coupled to a Perkin Elmer AutoSystem XL gas chromatograph equipped with a J&W Scientific DB1 capillary column (30 m, i.d. 0.45 mm, film 0.42 µm) and a Perkin Elmer TurboMass Upgrade MS detector. The volatiles were analyzed in the SIM mode using a two step temperature gradient starting from 60°C to 110°C at 2°C/min and then going to 260°C at 45°C/min. Headspace concentrations (in ng/L) were obtained by external standard calibrations as described in Example 5.

The following amounts of 2-phenylethanol were detected from the sample containing the stabilized hemiacetal as compared to the reference sample:

| | After 24 h [ng/L] | After 48 h [ng/L] |
|---|---|---|
| 2-Phenylethanol from stabilized hemiacetal | 1739.1 | 201.9 |
| 2-Phenylethanol (Reference) | 950.9 | 131.5 |

The data show that the sample containing the stabilized hemiacetals gives rise to higher headspace concentrations as compared to the reference sample. Stabilized hemiacetals are therefore suitable delivery systems for the controlled release of volatile alcohols in deodorant or antiperspirant applications.

## Claims

1. A dynamic mixture comprising a stabilized hemiacatal, said dynamic mixture being obtainable by reacting,
i) at least one active volatile alcohol having a molecular weight comprised between 42 and 230 g/mol and being a perfuming, flavoring, malodor counteracting, pharmaceutical, bactericide, fungicide, insect repellent or attractant ingredient;
with a composition comprising
ii) at least one mono, di or tri aldehyde of formula
HCO-Q (I)
wherein Q represents a CHO or (CH₂)ₘCOOH group, with m being 0, 1 or 2, or a group of formula 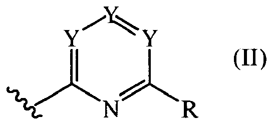 in which R represents a hydrogen atom, a CHO or COOH group or a C₁₋₆ hydrocarbon group, optionally comprising an oxygen or sulfur atom and optionally substituted:
each Y, simultaneously or independently, represents a CR group, R being defined as above, or a nitrogen atom, provided that two adjacent Y are not simultaneously nitrogen atoms; and two R groups, taken together, may form an unsaturated or aromatic cycle containing 5 or 6 atoms and including the carbon atoms to which said R is bonded, said cycle being substituted or unsubstituted and optionally containing one or two additional nitrogen or oxygen atoms; and,
iii) optionally, a stabilizing salt of formula MXₙ, a stabilizing acid having a pKₐ below 5, or a mixture thereof, wherein n represents 1, 2 or 3, X represents a mono-anion derived from an acid HX having a pKₐ below 6, and M is a cation selected from the group consisting of H⁺, Li⁺, Zn²⁺, Fe²⁺, Fe³⁺, Ni²⁺, Cu²⁺, Y³⁺, Sc³⁺, La³⁺, Ag⁺ and the alkaline-earth cations;
and wherein the substituents of said R comprise one or two groups such as R¹, OH, OR¹, SR¹, (NR²₃)R³, SO₂R⁴, SO₃R⁴, COOR⁴ or CHO, with R¹ representing a C₁-C₄, hydrocarbon group, or a phenyl group, optionally substituted by the same groups as defined above, R² representing a hydrogen atom or a R¹ group, R³ representing a halogen anion or a sulphate anion, R⁴ representing a hydrogen atom or an alkali metal cation.

2. A dynamic mixture according to claim 1, **characterized in that**:
the stabilizing salt is a compound MXₙ, wherein n represents 1, 2 or 3,
M is a cation selected from the group consisting of H⁺, Zn²⁺, Fe³⁺, Cu²⁺, Y³⁺, Sc³⁺, Ca²⁺, Mg²⁺ and Ba²⁺; and
X represents a mono-anion derived from an acid HX having a pKₐ below 5.

3. A dynamic mixture according to claim 1, **characterized in that** said aldehyde derivative is a compound of formula (III), (III') or (III") 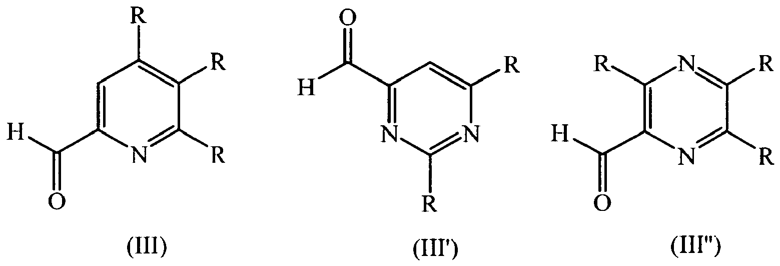 wherein R has the meaning defined in claim 1.

4. A dynamic mixture according to claim 1, **characterized in that** said aldehyde derivative is a
i) pyridine-2-carboxaldehyde, methyl 4-(6-formylpyridin-2-yl)benzoate, 6-(1,3-benzodioxol-5-yl)pyridine-2-carboxaldehyde, 4-(6-formylpyridin-2-yl)benzonitrile, 6-(4-(methylsulfonyl)phenyl)pyridine-2-carboxaldehyde, 6-(4-methylthio)phenyl)pyridine-2-carboxaldehyde, 6-(2- or 3-thienyl)pyridine-2-carboxaldehyde, quinoline-2-carboxaldehyde or pyridine-2,6-dicarboxaldehyde;
ii) pyrimidine-4-carboxaldehyde, pyrimidine-4,6-dicarboxaldehyde, 2-phenylpyrimidine-4,6-dicarboxaldehyde or pyrimidine-2,4,6-tricarboxaldehyde;
iii) pyrazine-2-carboxaldehyde, quinoxaline-2-carboxaldehyde or pyrazine-2,3- or 2,5- or 2,6-dicarboxaldehyde;
iv) oxoacetic acid;
and said derivatives of formula (I) being optionally substituted on the phenyl group, if present, by one to three methyl, methoxy, hydroxy or carboxylate groups.

5. A dynamic mixture according to claim 1, **characterized in that** said active volatile alcohol is a perfuming alcohol.

6. A dynamic mixture according to claim 5, **characterized in that** said perfuming alcohol is elected from the group consisting of primary, secondary or tertiary alcohols of formula R⁷OH, wherein R⁷ represents a C₄-C₁₅ saturated or unsaturated, linear or branched, mono- or bicyclic hydrocarbon group, optionally substituted with one, two or three C₁-C₃ alkyl or alkenyl, methoxy, ethoxy groups and/or phenyl groups optionally substituted with one, two or three C₁-C₃ alkyl or alkenyl, methoxy, ethoxy groups.

7. Use as active ingredient of a dynamic mixture as defined in any one of claims 1 to 6.

8. A perfuming composition comprising:
i) as perfuming ingredient, a dynamic mixture as defined in claim 1;
ii) at least one ingredient selected from the group consisting of a perfumery carrier and a perfumery base; and
iii) optionally at least one perfumery adjuvant.

9. A consumer article comprising
i) as active ingredient, a dynamic mixture as defined in claim 1: and
ii) a consumer product base.

10. A consumer article according to claim 9, **characterized in that** said product is a perfumed or flavored product.

11. A consumer article according to claim 9, **characterized in that** said product is a solid or liquid detergent, fabric softener, hygiene product, body-care product or cosmetic.

12. A consumer article according to claim 9, **characterized in that** said product is a perfume, cologne or after-shave lotion, a perfumed soap, shower or bath mousse, oil or gel, or a hair care product such as a shampoo or a hair spray, a deodorant or antiperspirant, an air freshener or a "ready to use" powdered air freshener, or a detergent, or a fabric refresher, an ironing water, a paper, a wipe or a bleach.

## Patentansprüche

1. Dynamische Mischung, umfassend ein stabilisiertes Halbacetal, wobei die dynamische Mischung erhältlich ist durch Umsetzen von
i) mindestens einem aktiven flüchtigen Alkohol, der ein Molekulargewicht zwischen 42 und 230 g/mol aufweist und ein parfümierender, aromatisierender, üblem Geruch entgegenwirkender, pharmazeutischer, bakterizider, fungizider, Insekten vertreibender oder anlockender Bestandteil ist;
mit einer Zusammensetzung, umfassend
ii) mindestens ein Mono-, Di- oder Trialdehyd der Formel
HCO-Q (I)
worin Q eine CHO- oder (CH₂)ₘCOOH-Gruppe, wobei m 0, 1 oder 2 ist, oder eine Gruppe der Formel 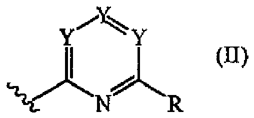 darstellt, in welcher R ein Wasserstoffatom, eine CHO- oder COOH-Gruppe, oder eine C₁₋₆-Kohlenwasserstoffgruppe, wahlweise umfassend ein Sauerstoff- oder Schwefelatom und wahlweise substituiert, darstellt;
jedes Y, gleichzeitig oder unabhängig, eine CR-Gruppe, wobei R wie vorstehend definiert ist, oder ein Stickstoffatom darstellt, mit der Maßgabe, dass zwei benachbarte Y nicht gleichzeitig Stickstoffatome sind; und zwei R-Gruppen, zusammengenommen, einen ungesättigten oder aromatischen Cyclus, enthaltend 5 oder 6 Atome und einschließend die Kohlenstoffatome, an welche das R gebunden ist, bilden können, wobei der Cyclus substituiert oder unsubstituiert ist und wahlweise ein oder zwei zusätzliche Stickstoff- oder Sauerstoffatome enthält; und
iii) wahlweise, ein stabilisierendes Salz der Formel MXₙ, eine stabilisierende Säure mit einem pKₐ unter 5, oder eine Mischung davon, wobei n 1, 2 oder 3 darstellt, X ein Monoanion, abgeleitet von einer Säure HX mit einem pKₐ unter 6, darstellt und M ein Kation, ausgewählt aus der Gruppe, bestehend aus H⁺, Li⁺, Zn²⁺, Fe²⁺, Fe³⁺, Ni²⁺, Cu²⁺, Y³⁺, Sc³⁺, La³⁺, Ag⁺ und den Erdalkalikationen, ist;
und worin die Substituenten des R eine oder zwei Gruppen, wie beispielsweise R¹, OH, OR¹, SR¹, (NR²₃)R³, SO₂R⁴, SO₃R⁴, COOR⁴ oder CHO, umfassen, wobei R¹ eine C₁-C₄-Kohlenwasserstoffgruppe oder eine Phenylgruppe, wahlweise substituiert durch die gleichen Gruppen wie vorstehend definiert, darstellt, wobei R² ein Wasserstoffatom oder eine R¹-Gruppe darstellt, wobei R³ ein Halogenanion oder ein Sulfatanion darstellt, wobei R⁴ ein Wasserstoffatom oder ein Alkalimetallkation darstellt.

2. Dynamische Mischung gemäß Anspruch 1, **dadurch gekennzeichnet, dass**:
das stabilisierende Salz eine Verbindung MXₙ ist, worin n 1, 2 oder 3 darstellt,
M ein Kation, ausgewählt aus der Gruppe, bestehend aus H⁺, Zn²⁺, Fe³⁺, Cu²⁺, Y³⁺, Sc³⁺, Ca²⁺, Mg²⁺ und Ba²⁺, ist; und
X ein Monoanion, abgeleitet von einer Säure HX mit einem pKₐ unter 5, darstellt.

3. Dynamische Mischung gemäß Anspruch 1, **dadurch gekennzeichnet, dass** das Aldehyd-Derivat eine Verbindung der Formel (III), (III') oder (III") 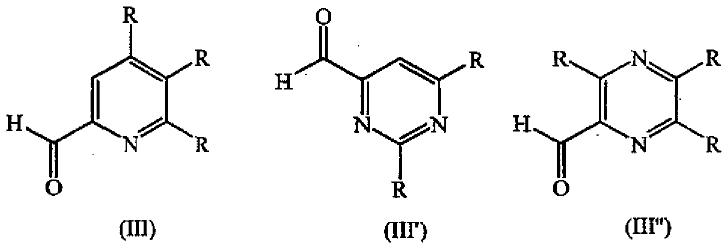 ist, worin R die Bedeutung, definiert in Anspruch 1, hat.

4. Dynamische Mischung gemäß Anspruch 1, **dadurch gekennzeichnet, dass** das Aldehyd-Derivat ein
i) Pyridin-2-carboxaldehyd, Methyl-4-(6-formylpyridin-2-yl)benzoat, 6-(1,3-Benzodioxol-5-yl)pyridin-2-carboxaldehyd, 4-(6-Formylpyridin-2-yl)benzonitril, 6-(4-(Methylsulfonyl)phenyl)pyridin-2-carboxaldehyd, 6-(4-Methylthiophenyl)pyridin-2-carboxaldehyd, 6-(2- oder 3-Thienyl)pyridin-2-carboxaldehyd, Chinolin-2-carboxaldehyd oder Pyridin-2,6-dicarboxaldehyd;
ii) Pyrimidin-4-carboxaldehyd, Pyrimidin-4,6-dicarboxaldehyd, 2-Phenylpyrimidin-4,6-dicarboxaldehyd oder Pyrimidin-2,4,6-tricarboxaldehyd;
iii) Pyrazin-2-carboxaldehyd, Chinoxalin-2-carboxaldehyd oder Pyrazin-2,3-oder -2,5- oder -2,6-Dicarboxaldehyd;
iv) Oxoessigsäure;
ist, und wobei die Derivate der Formel (I) wahlweise an der Phenylgruppe, wenn vorhanden, durch eine bis drei Methyl-, Methoxy-, Hydroxy- oder Carboxylatgruppen substituiert sind.

5. Dynamische Mischung gemäß Anspruch 1, **dadurch gekennzeichnet, dass** der aktive flüchtige Alkohol ein parfümierender Alkohol ist.

6. Dynamische Mischung gemäß Anspruch 5, **dadurch gekennzeichnet, dass** der parfümierende Alkohol aus der Gruppe, bestehend aus primären, sekundären oder tertiären Alkoholen der Formel R⁷OH, ausgewählt ist, wobei R⁷ eine C₄-C₁₅-gesättigte oder ungesättigte, lineare oder verzweigte, mono- oder bicyclische Kohlenwasserstoffgruppe, wahlweise substituiert mit einer, zwei oder drei C₁-C₃-Alkyl-oder -Alkenyl-, Methoxy-, Ethoxygruppen und/oder Phenylgruppen, wahlweise substituiert mit einer, zwei oder drei C₁-C₃-Alkyl- oder -Alkenyl-, Methoxy-, Ethoxygruppen, darstellt.

7. Verwendung einer dynamischen Mischung, wie definiert in einem der Ansprüche 1 bis 6, als aktiver Bestandteil.

8. Parfümierende Zusammensetzung, umfassend:
i) als parfümierenden Bestandteil eine dynamische Mischung, wie definiert in Anspruch 1;
ii) mindestens einen Bestandteil, ausgewählt aus der Gruppe, bestehend aus einem Träger für Parfümfabrikation und einer Basis für Parfümfabrikation; und
iii) wahlweise mindestens einen Hilfsstoff für Parfümfabrikation.

9. Verbrauchergegenstand, umfassend
i) als aktiven Bestandteil eine dynamische Mischung, wie definiert in Anspruch 1; und
ii) eine Verbraucherproduktbasis.

10. Verbrauchergegenstand gemäß Anspruch 9, **dadurch gekennzeichnet, dass** das Produkt ein parfümiertes oder aromatisiertes Produkt ist.

11. Verbrauchergegenstand gemäß Anspruch 9, **dadurch gekennzeichnet, dass** das Produkt ein festes oder flüssiges Detergens, ein Weichspüler, ein Hygieneprodukt, ein Körperpflegeprodukt oder Kosmetikum ist.

12. Verbrauchergegenstand gemäß Anspruch 9, **dadurch gekennzeichnet, dass** das Produkt ein Parfüm, ein Kölnischwasser oder eine Aftershavelotion, eine parfümierte Seife, ein Dusch- oder Badeschaum, -öl oder -gel, oder ein Haarpflegeprodukt wie beispielsweise ein Shampoo oder ein Haarspray, ein Deodorant oder Antiperspirant, ein Luftverbesserer oder ein "gebrauchsfertiger" pulverförmiger Luftverbesserer, oder ein Detergens, oder ein Gewebeauffrischer, ein Bügelwasser, ein Papier, ein Wischtuch oder ein Bleichmittel ist.

## Revendications

1. Mélange dynamique comprenant un hémiacétal stabilisé, ledit mélange dynamique pouvant être obtenu par réaction de
i) au moins un alcool volatil actif ayant une masse moléculaire comprise entre 42 et 230 g/mol et qui est un ingrédient parfumant, aromatisant, neutralisant les mauvaises odeurs, pharmaceutique, bactéricide, fongicide, insectifuge ou appât pour insectes;
avec une composition comprenant
ii) au moins un mono, di ou trialdéhyde de formule
HCO-Q (I)
dans laquelle Q représente un groupe CHO ou (CH₂)ₘCOOH, m valant 0, 1 ou 2, ou un groupe de formule 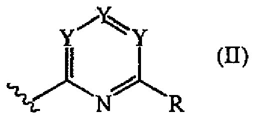 dans laquelle R représente un atome d'hydrogène, un groupe CHO ou COOH ou un groupe hydrocarboné en C₁₋₆, comprenant optionnellement un atome d'oxygène ou de soufre et optionnellement substitué;
chaque Y, simultanément ou indépendamment, représente un groupe CR, R étant défini comme ci-dessus, ou un atome d'azote, à condition que deux Y adjacents ne soient pas simultanément des atomes d'azote; et deux groupes R, pris ensemble, peuvent former un cycle insaturé ou aromatique contenant 5 ou 6 atomes y compris les atomes de carbone auxquels ledit R est lié, ledit cycle étant substitué ou non substitué et contenant optionnellement un ou deux atomes d'azote ou d'oxygène supplémentaires; et
iii) optionnellement, un sel stabilisant de formule MXₙ, un acide stabilisant ayant un pKₐ inférieur à 5, ou de leurs mélanges, où n représente 1, 2 ou 3, X représente un mono-anion dérivé d'un acide HX ayant un pKₐ inférieur à 6, et M est un cation choisi dans le groupe constitué de H⁺, Li⁺, Zn²⁺, Fe²⁺, Fe³⁺, Ni²⁺, Cu²⁺, Y³⁺, Sc³⁺, La³⁺, Ag⁺ et des cations alcalino-terreux;
et dans laquelle les substituants dudit R comprennent un ou deux groupes tels que R¹, OH, OR¹, SR¹, (NR²₃)R³, SO₂R⁴, SO₃R⁴, COOR⁴ ou CHO, R¹ représentant un groupe hydrocarboné en C₁-C₄, ou un groupe phényle, optionnellement substitué par les mêmes groupes que ceux définis ci-dessus, R² représentant un atome d'hydrogène ou un groupe R¹, R³ représentant un anion halogène ou un anion sulfate, R⁴ représentant un atome d'hydrogène ou un cation de métal alcalin.

2. Mélange dynamique selon la revendication 1, **caractérisé en ce que**:
le sel stabilisant est un composé MXₙ, où n représente 1, 2 ou 3,
M est un cation choisi dans le groupe constitué de H⁺, Zn²⁺, Fe³⁺, Cu²⁺, Y³⁺, Sc³⁺, Ca²⁺, Mg²⁺ et Ba²⁺; et
X représente un mono-anion dérivé d'un acide HX ayant un pKₐ inférieur à 5.

3. Mélange dynamique selon la revendication 1, **caractérisé en ce que** ledit
dérivé d'aldéhyde est un composé de formule (III), (III') ou (III") 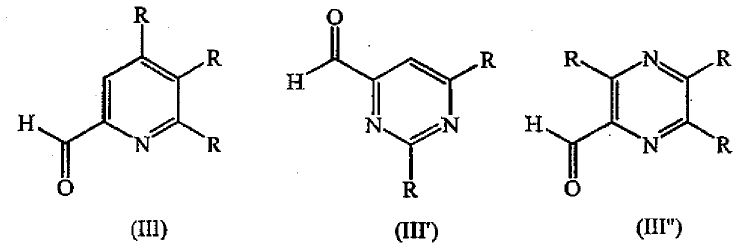 où R a la signification donnée dans la revendication 1.

4. Mélange dynamique selon la revendication 1, **caractérisé en ce que** ledit dérivé d'aldéhyde est un
i) pyridine-2-carboxaldéhyde, 4-(6-formylpyridin-2-yl)benzoate de méthyle, 6-(1,3-benzodioxol-5-yl)pyridine-2-carboxaldéhyde, 4-(6-formylpyridin-2-yl)benzonitrile, 6-(4-(méthylsulfonyl)phényl)pyridine-2-carboxaldéhyde, 6-(4-(méthylthio)phényl)pyridine-2-carboxaldéhyde, 6-(2- ou 3-thiényl)pyridine-2-carboxaldéhyde, quinoléine-2-carboxaldéhyde ou pyridine-2,6-dicarboxaldéhyde;
ii) pyrimidine-4-carboxaldéhyde, pyrimidine-4,6-dicarboxaldéhyde, 2-phénylpyrimidine-4,6-dicarboxaldéhyde ou pyrimidine-2,4,6-tricarboxaldéhyde;
iii) pyrazine-2-carboxaldéhyde, quinoxaline-2-carboxaldéhyde ou pyrazine-2,3- ou 2,5- ou 2,6-dicarboxaldéhyde;
iv) acide oxoacétique;
et lesdits dérivés de formule (I) étant optionnellement substitués sur le groupe phényle, s'il est présent, par un à trois groupes méthyle, méthoxy, hydroxy ou carboxylate.

5. Mélange dynamique selon la revendication 1, **caractérisé en ce que** ledit alcool volatil actif est un alcool parfumant.

6. Mélange dynamique selon la revendication 5, **caractérisé en ce que** ledit alcool parfumant est choisi dans le groupe constitué des alcools primaires, secondaires ou tertiaires de formule R⁷OH, dans laquelle R⁷ représente un groupe hydrocarboné mono- ou bicyclique, linéaire ou ramifié, saturé ou insaturé, en C₄-C₁₅, optionnellement substitué par un, deux ou trois groupes alkyle ou alcényle en C₁-C₃, méthoxy, éthoxy et/ou groupes phényle optionnellement substitués par un, deux ou trois groupes groupes alkyle ou alcényle en C₁-C₃, méthoxy, éthoxy.

7. Utilisation, en tant qu'ingrédient actif, d'un mélange dynamique tel que défini dans l'une quelconque des revendications 1 à 6.

8. Composition parfumante comprenant:
i) en tant qu'ingrédient parfumant, un mélange dynamique tel que défini dans la revendication 1;
ii) au moins un ingrédient choisi dans le groupe constitué d'un véhicule de parfumerie et d'une base de parfumerie; et
iii) optionnellement, au moins un adjuvant d'usage courant en parfumerie.

9. Article de consommation comprenant:
i) en tant qu'ingrédient actif, un mélange dynamique tel que défini dans la revendication 1 ; et
ii) une base de produit de consommation.

10. Article de consommation selon la revendication 9, **caractérisé en ce que** ledit produit est un produit parfumé ou aromatisé,

11. Article de consommation selon la revendication 9, **caractérisé en ce que** ledit produit est un détergent solide ou liquide, un adoucissant pour textile, un produit d'hygiène, un produit de soin corporel ou une préparation cosmétique.

12. Article de consommation selon la revendication 9, **caractérisé en ce que** ledit produit est un parfum, une eau de Cologne ou une lotion après-rasage, un savon parfumé, une mousse, une huile ou un gel de bain ou de douche, ou un produit de soin capillaire tel qu'un shampooing ou une laque capillaire, un déodorant ou un antitranspirant, un désodorisant d'air ambiant ou un désodorisant d'air ambiant en poudre "prêt à l'emploi", ou un détergent, ou une eau de linge, une eau de repassage, un papier, une lingette ou un agent de blanchiment.
